# EUROPEAN PATENT APPLICATION

(11) **EP 2 574 608 A1**
(43) Date of publication of application: **03.04.2013**
(21) Application number: 11007868.0
(22) Date of filing: 28.09.2011
(51) Int. Cl.: C07D 265/38, C09K 11/00, H01L 51/00

(54) **Spirobifluorene compounds for light emitting devices**

(71) Applicant: SOLVAY SA, 1120 Bruxelles (BE)
(72) Inventor: The designation of the inventor has not yet been filed
(74) Representative: Langfinger, Klaus Dieter

(57) **Abstract**

Novel spirobifluorene compounds for light emitting devices where the spirobifluorene ring system comprises at least one ligand in meta-position which is bound to the spirobifluorene through a heteroatom not being part of a ring system

## Description

The present invention relates to compounds based on spirobifluorene and light emitting devices comprising said compounds.

Various organic light emitting devices have been under active study and development, particularly those based on electroluminescence (EL) from small organic materials. For such organic devices, the ability to form morphologically stable amorphous films is a key requirement for the development of small materials for organic light emitting diodes (OLEDs). That is because when a small molecule compound is used in the organic light-emitting layer, crystallization usually occurs if the molecule of the compound is too small and its structure is too symmetrical. Therefore, when applied in an organic emission layer, the small molecule compound is vulnerable to morphological change such as crystallization, and once the crystal is formed, it yields negative impacts upon the light-emitting nature and service life of the OLED.

Thermal stress during device operation can lead to such phase transitions from the amorphous state to the thermodynamically stable polycrystalline state leading to dramatic degradation of the device. As a result it is crucial to design materials featuring high glass transition temperature (Tg >150°C) in order to stabilize the amorphous state. For improving the stability of devices in order to increase operational lifetime, several host materials have been reported. Especially, designing materials having a spiro linkage has been a very successful strategy to obtain OLEDs materials with enhanced morphological stability while keeping their electro-optical functionality.

US2006/0141287 discloses light-emitting layers which include a solid organic material containing a mixture of at least two components. The first host component is an organic compound capable of transporting electrical charges and also forms an aggregate. The second component of the mixture is an organic compound capable of transporting electrical charges and, upon mixing with the first host component, is capable of forming a continuous and substantially pin-hole-free layer. In the reference, as the second component, various compounds such as substituted fluorene derivatives, and spirobifluorene derivatives, etc. are used.

Spirobifluorene, as used herein denotes a structural element of formula (1) and is referred to as SBF hereinafter, whereas Open SBF denotes a system of formula (2) below.

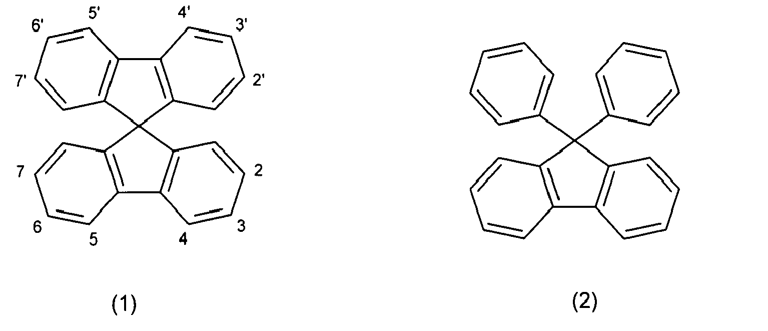

Substituted spirobifluorene compounds have been extensively described in the prior art, in particular with a substitution of the SBF ring system in "para-position (i.e. the 2, 7, 2' or 7' position of the SBF) by a heteroatom.

Substituted spirobifluorene of this type where the heteroatom bound to the spirobifluorene unit is part of a ring system have also been described.

Salbeck et al., Chem.Rev. 2007, 107, 1011-1065 provides a good overview of spiro compounds useful in organic optoelectronics. Thus, compounds (16) and (17) in Salbeck are the following

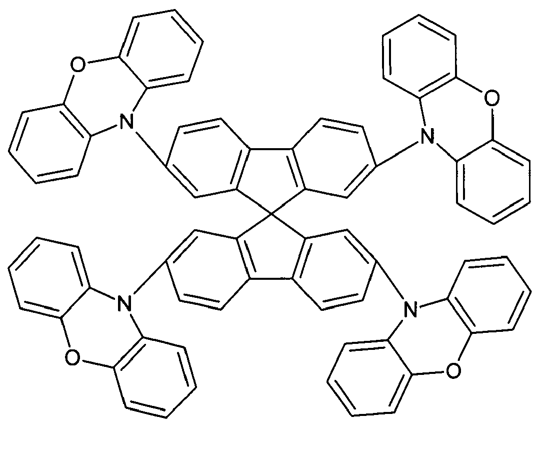

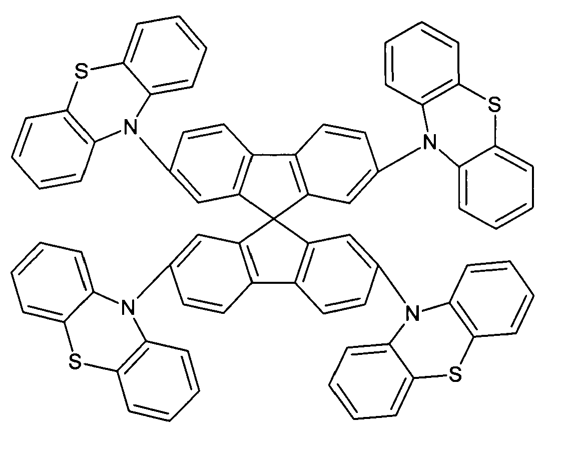

These compounds are reported to have absorption maxima below 400 nm and emission maxima of 491 and 511 nm, respectively. No data on efficiency are given.

Salbeck further reports that replacement of diphenylamino substituents like in the compounds above by carbazole ligands results in a distinct hypsochromic shift of absorption and emission. As an example the compound where the substitutents are replaced by N-carbazole shows an absorption maximum below 350 nm and an emission maximum at 372 nm.

Spirobifluorene compounds with diphenylamino substituents in para-postion of the SBF unit are again disclosed in Salbeck et al., see e.g. compounds 42 to 48 thereof.

WO 2011/06574 discloses 4 and 4,4'diphenylamino-substituted SBF compounds (which may be referred to as ortho-substituted compounds relative to the direct bond linking the phenyl rings of the SBF unit).

European Patent Application 2 312 667 discloses compositions for organic electroluminescence elements comprising at least two different materials fulfilling a certain mathematical equation related to the solubility of the materials. Among an extended list of suitable materials having as a common structural feature substituted diphenylamino groups, 3,6-Bis-N,N'-di(4-tert.butylphenyl)amino-spirobifluorene as well as the respective Open SBF derivative are mentioned.

None of the above-disclosed materials meets all the requirements necessary for OLED application, particularly suitable energy level for high phosphorescent efficiency (high triplet energy), high morphological stability, while maintaining other electro-optic and processing properties under operational conditions of the device, such as emission color, dimensional stability, etc, in a fully satisfactory manner. Thus, there has been a need to develop new host materials, which are capable of satisfying all of the requirements indicated above.

Surprisingly, it has been found that spirobifluorene compounds as defined in claim 1 possess a property spectrum which makes them particularly suitable for use in organic electroluminescent devices.

Preferred compounds in accordance with the instant invention are described in the dependent claims and the detailed specification hereinafter.

The compounds of the present invention are characterized by formulae 1 to 12 below

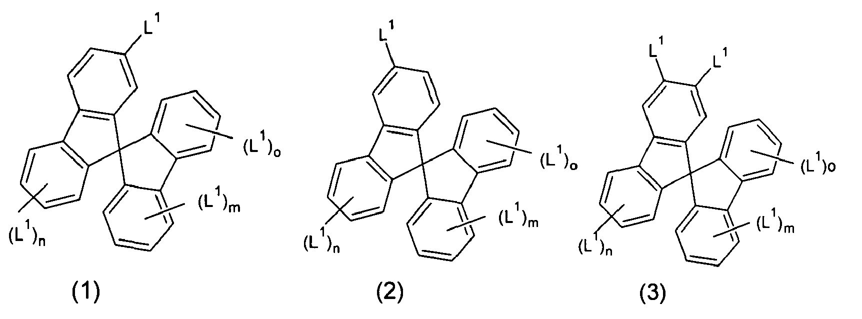

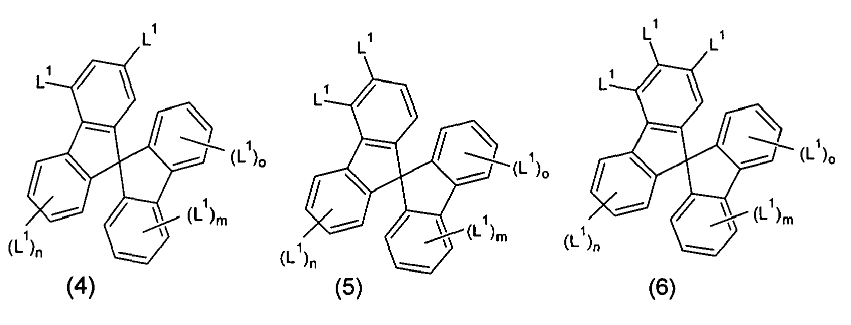

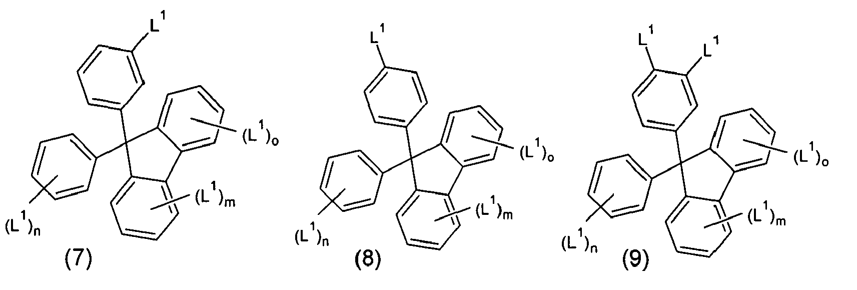

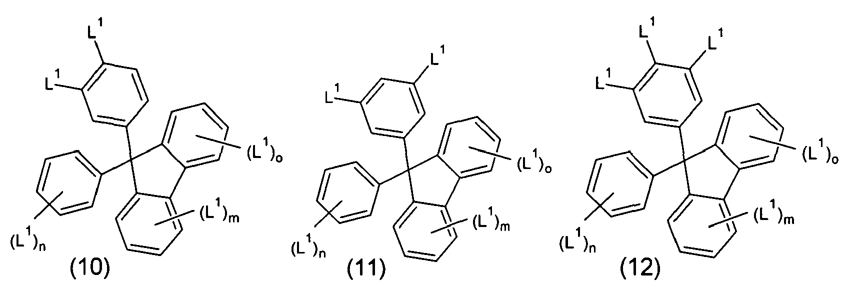

wherein n, m and o may be the same or different and represent an integer of from 0 to 3 with the proviso that in compounds of formula 1 at least one of n, m or o is zero,
each of the phenyl rings may carry no ligands other than L¹ or may be substituted by ligands other than L¹,

L¹, which may be the same or different in each position, has the formula A

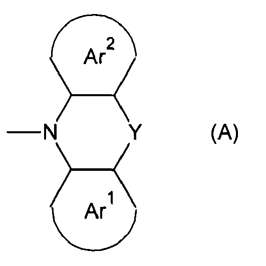

wherein

Y is selected from the group consisting of O, S, C=O, -CR¹R², N-R³, S=O, S(=O)₂, PR⁴ and P(=O)R⁵,

R¹ to R⁵ may be the same or different in each position and represent hydrogen or an aliphatic, carbocyclic, aromatic or heterocyclic group with 1 to 20 carbon atoms, and

Ar¹ and Ar² represent optionally substituted aromatic or heteroaromatic ring systems comprising 4 to 20 ring atoms (the two ring atoms of the heterocyclic ring shown in formula I being part of the aromatic or heteroaromatic ring system for the purpose of counting ring atoms).

The compounds in accordance with the present invention share the common feature that the SBF or Open SBF unit is substituted by a nitrogen atom, which is part of a ring system comprising two aromatic or heteroaromatic rings.

The substitution in the SBF system may be para, meta or ortho to the bond linking the phenyl rings in the SBF unit or in the analogous positions of the Open SBF unit.

It has been found that for certain purposes compounds having at least one substituent L¹ in meta position can be advantageous in terms of efficiency when used in organic electronic devices.

A first preferred group of compounds are those where L¹ has the formula A1

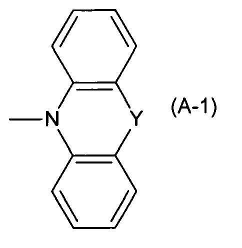

where Y is as defined above.

Y is preferably selected from the group consisting of O, S, C=O, CR¹R², N-R³ and S=O, particularly preferred from O, S and N-R³, even more preferably from O or S, most preferably Y is O, wherein R1 to R³ have the meaning as defined above.

If R¹ to R⁵ represents an alkyl group, same has preferably 1 to 20, especially 1 to 8 carbon atoms and may be straight chain or branched. Particularly preferred alkyl groups are C₁ to C₄ alkyl like methyl, ethyl, i- or n-propyl and i-, n- and t-butyl. The alkyl groups may themselves be substituted or unsubstituted.

Preferred carbocyclic groups for R¹ to R⁵ are 5 to 7 membered carbocyclic ring systems, which may be saturated or unsaturated like e.g. cyclopentane, cyclohexane or cyclohexene, to give only three examples. As for the alkyl groups, the carbocyclic groups may be substituted or unsubstituted.

Preferred aryl groups for R¹ to R⁵ are are phenyl, napththyl, anthracenyl, biphenyl or terphenyl, which may be unsubstituted or substituted by substituents selected from the group consisting of halogen, alkyl, alkoxy, amino, cyano, alkenyl, alkynyl, arylalkyl, aryl and heteroaryl groups or the aryl group may be part of an annealed ring system.

Especially preferred aryl substituents are derived from the following substituted or unsubstituted aryl systems

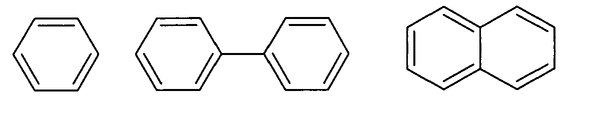

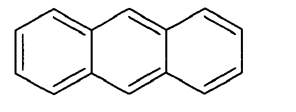

of which phenyl and biphenyl are especially preferred.

A particularly preferred group of heteroaryl groups for R¹ to R⁵ are the following:

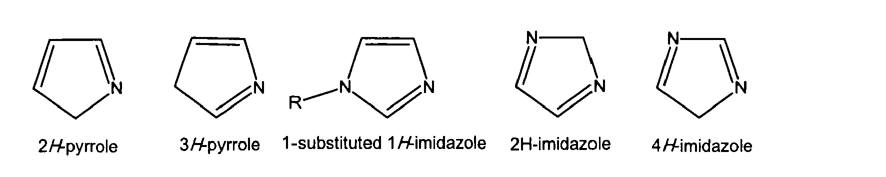

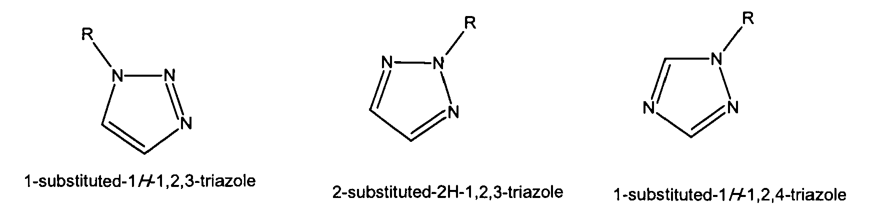

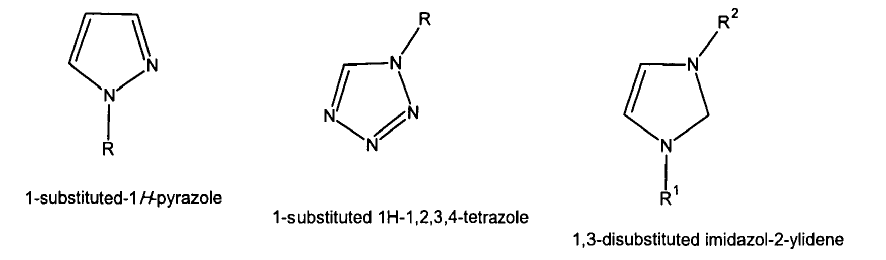

In all these ring systems one or more of the nitrogen atoms may be replaced by another heteroatom like O or S, to name only two examples as shown below:

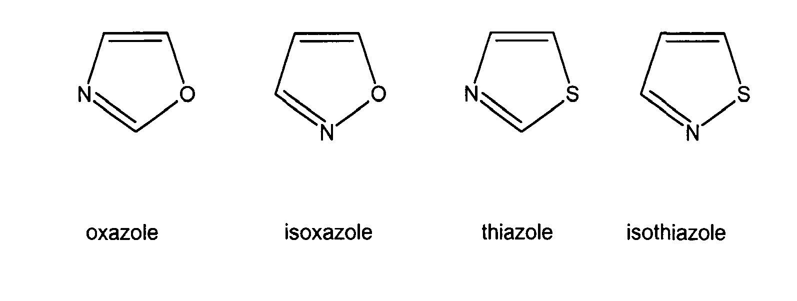

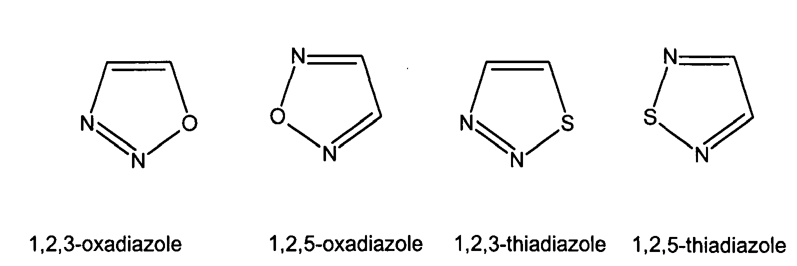

Still another preferred group of heteroaryl substituents comprises the 6-membered ring systems shown below:

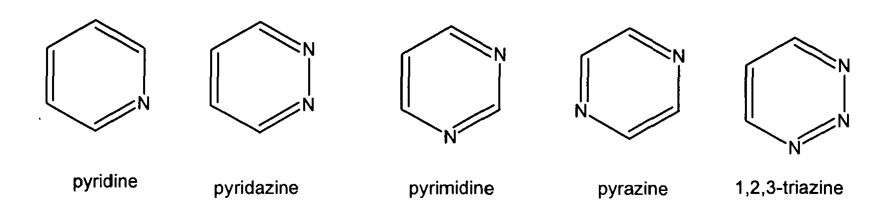

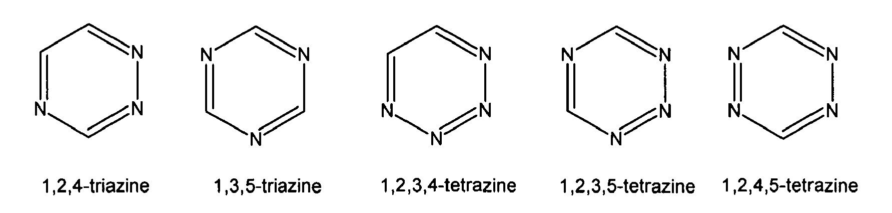

In the same manner as the aryl groups the aforementioned heteroaryl groups may be substituted, preferably by substituents selected from the group consisting of halogen, alkyl, alkoxy, amino, cyano, alkenyl, alkynyl, arylalkyl, aryl and heteroaryl groups or the heteroaryl group may be part of an annealed ring system.

It is apparent to the skilled person that steric reasons may exclude or render difficult a certain combination of R¹ and R² in the compounds of the present invention, no further explanations n this regard being necessary here.

Ar¹ and Ar², which may be the same or different can be selected from the aromatic or heteroaromatic ring systems described above for substitutents R¹ to R⁵ and thus reference thereto is made at this point. Preferably, Ar¹ and or Ar², which may be the same or different, are aryl ring systems as defined above, preferably phenyl or naphthyl, which may be substituted or unsubstituted.

A further group of preferred compounds in accordance with the present invention are those wherein at least one of n, m or o represents an integer of from 1 to 3.

Also preferred are compounds wherein n, m and o are all zero.

Further preferred amongst such compounds (where n, m and o are zero are compounds of general formula

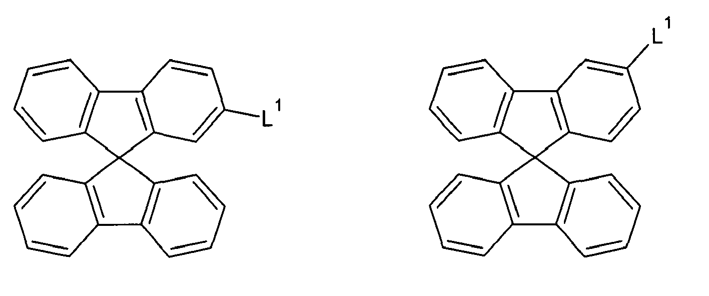

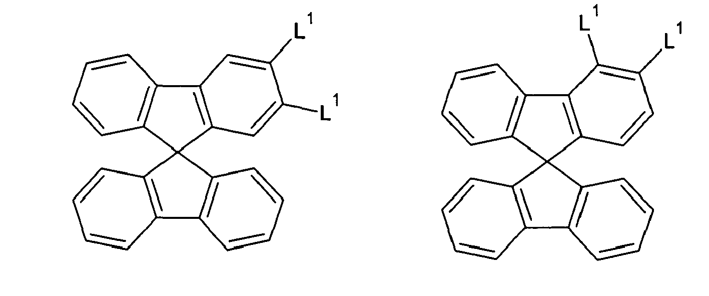

where L¹ can have any of the meanings defined above.

Accordingly a particularly preferred group of compounds of the formulae above carries substituents of formula A-1 and in particular substituents A-1 wherein Y is O or S.

The SBF or open SBF ring system may or may not comprise further substituents in addition to substituents L¹. If present, such additional substituents, which may be the same or different in each position they occur, are generally selected from of halogen, alkyl, alkoxy, amino, cyano, alkenyl, alkynyl, arylalkyl, aryl and heteroaryl groups.

The compounds in accordance with the present invention may be synthesized by any known and suitable method. The skilled person is aware of suitable manufacturing processes.

Generally, the compounds of the present invention with meta-substituents may be prepared by the following general reaction schemes, which show an exemplary way for compounds carrying one or two ligands L¹

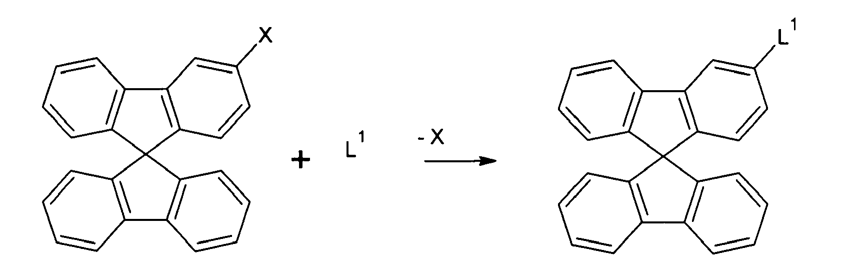

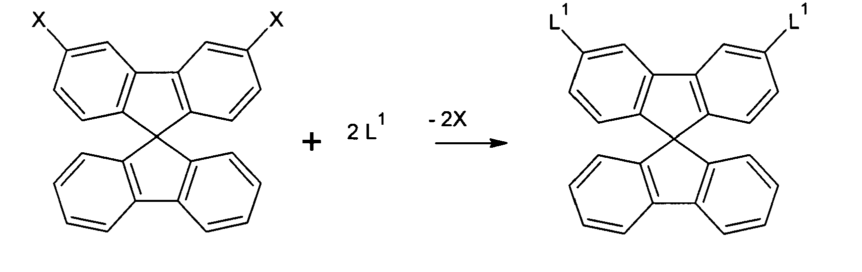

wherein X is a leaving group selected from known leaving groups for such reactions such as halogen, OH, OR, SR , OCN, SCN or CN, especially preferably halogen, in particular chlorine or bromine.

The skilled person will select the suitable reactants and reaction conditions based on the individual needs of a specific synthesis.

The starting materials for such synthesis with at least one leaving group in a meta-position of the SBF or Open SBF ring system may be synthesized in accordance with various process routes which the skilled person will select in accordance with the specific needs. Generally, such compounds are not easily accessible through introduction of the substituents directly into a SBF or Open SBF core as these routes generally yield the para-substituted products preferably due to their higher reactivity. Accordingly, the substituents X have to be introduced through suitable precursor substances e.g. fluorene derivatives, benzophenone derivatives or biphenyl derivatives, to mention only three examples, which are thereafter reacted to yield the SBF or Open SBF structure.

Thus respective compounds may for example be obtained from substituted fluorenone derivatives of formula

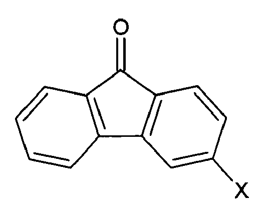

with suitable biphenyl compounds.

Another possibility is the reaction of fluorenones with suitable substituted biphenyl compounds in accordance with the general reaction scheme

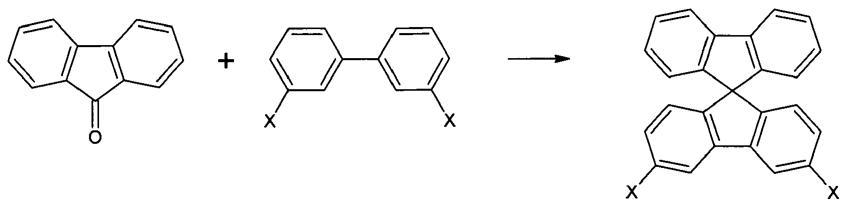

which is described in more detail in JP 2006/089585 for X= OH and which may be adopted for other substituents X.

Another embodiment of the present invention is directed to the use of the compounds of the present invention in an organic light emitting device, especially an organic light emitting diode (OLED).

The compounds in accordance with the present invention may advantageously be used, together with an emitting material, in the emissive layer of an organic light emitting device.

The present invention is also directed to an organic light emitting device (OLED) comprising an emissive layer (EML), said emissive layer comprising the compounds of the present invention as host material, said host material being notably suitable in an emissive layer (EML) in an OLED.

An OLED generally comprises :
a substrate, for example (but not limited to) glass, plastic, metal;
an anode, generally transparent anode, such as an indium-tin oxide (ITO) anode;
a hole injection layer (HIL) for example (but not limited to) PEDOT/PSS;
a hole transporting layer (HTL);
an emissive layer (EML);
an electron transporting layer (ETL);
an electron injection layer (EIL) such as LiF, Cs₂CO₃
a cathode, generally a metallic cathode, such as an Al layer.

For a hole conducting emissive layer, one may have a hole blocking layer (HBL) that can also act as an exciton blocking layer between the emissive layer and the electron transporting layer. For an electron conducting emissive layer, one may have an electron blocking layer (EBL) that can also act as an exciton blocking layer between the emissive layer and the hole transporting layer. The emissive layer may be equal to the hole transporting layer (in which case the exciton blocking layer is near or at the anode) or to the electron transporting layer (in which case the exciton blocking layer is near or at the cathode).

The compounds of the present invention may be used preferably used as hosts in an emissive layer.

Optionally, the emissive layer may also contain a polarization molecule, present as a dopant in said host material and having a dipole moment that generally affects the wavelength of light emitted.

A layer formed of an electron transporting material is advantageously used to transport electrons into the emissive layer comprising the light emitting material and the (optional) host material. The electron transporting material may be an electron-transporting matrix selected from the group of metal quinoxolates (e.g. Alq₃, Liq), oxadiazoles, triazoles and ketones (e.g. Spirobifluorene ketones SBFK). Examples of electron transporting materials are tris-(8-hydroxyquinoline)aluminum of formula ["Alq₃"] and spirobifluoreneketone SBFK:

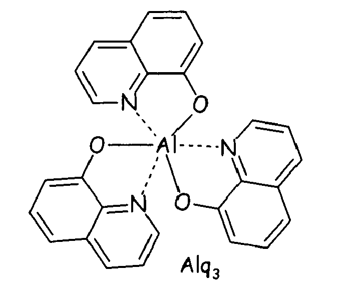

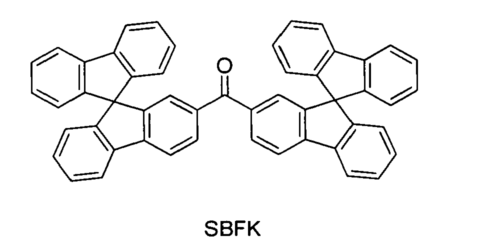

A layer formed of a hole transporting material is advantageously used to transport holes into the emissive layer comprising the light emitting material as above described and the (optional) host material. An example of a hole transporting material is 4,4'-bis[N-(1-naphthyl)-N-phenylamino]biphenyl ["α-NPD"].

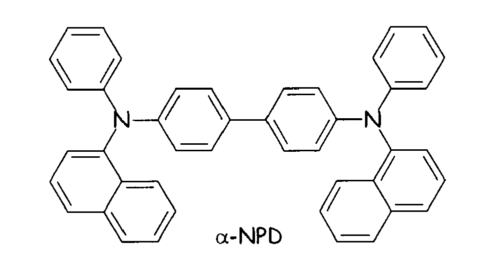

The use of an exciton blocking layer ("barrier layer") to confine excitons within the luminescent layer ("luminescent zone") is greatly preferred. For a hole-transporting host, the blocking layer may be placed between the emissive layer and the electron transport layer. An example of a material for such a barrier layer is 2,9-dimethyl-4,7-diphenyl-1,10-phenanthroline (also called bathocuproine or "BCP"), which has the formula

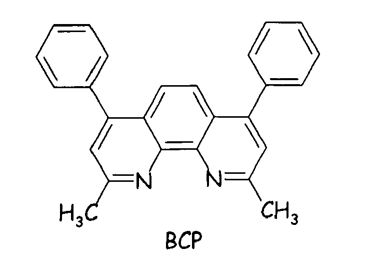

### BCP

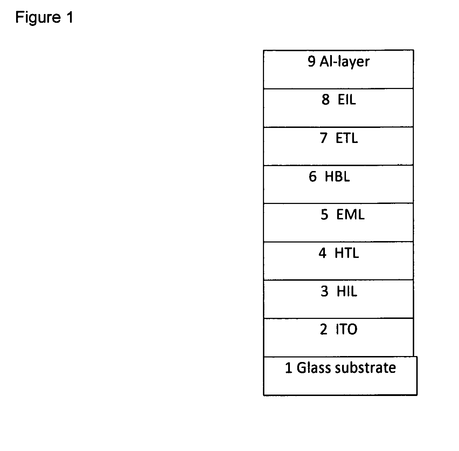

The OLED has preferably a multilayer structure, as depicted in Figure 1, wherein 1 is a glass substrate, 2 is an ITO layer, 3 is a HIL layer comprising PEDOT/PSS, 4 is a HTL layer comprising α-NPD, 5 is an EML comprising mCBP as host material and the light emitting material or mixture of these materials as above defined as dopant in an amount of about 15 % wt with respect to the total weight of host plus dopant; 6 is a HBL comprising BCP; 7 is an ETL comprising Alq₃; 8 is an EIL comprising LiF and 9 is an Al layer cathode.

An example of the present invention is reported hereinafter, whose purpose is merely illustrative but not limitative of the scope of the invention itself.

Example 1

Synthesis of 3-Bromo-SBF

Step 1: Synthesis of 3-Bromofluorenone

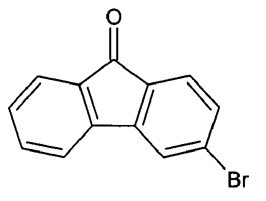

In a three ways flask 60 ml of water were added to 8.9 ml of hydrochloric acid (HCl, 37 % w/w, 2.1 molar equivalents) and the medium was cooled to 0°C. NaNO₂ (1.5 molar equivalents), dissolved in 50 ml of water, was added dropwise at 0°C. At the end of the addition, 4-amino-2-bromobenzophenone (one equivalent, 15.0 g, 51.6 mmole) solubilised in a mixture of acetone/water (400/230 ml), was added carefully. After 30 minutes at room temperature, the mixture was warmed up and kept at 60 °C for 3 hours.

After extraction with methylene chloride and evaporation of the organic phase, a brown solid was recovered (17.4 g) and a flash chromatography is realized. The pure compound was recovered after crystallization with hexane (4.2 g, 32 % yield).

Step 2: 3-bromo-SBF

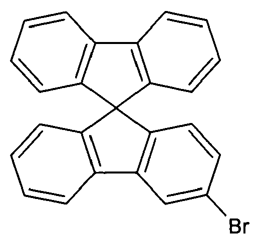

This compound was made in two steps from 3-bromofluorenone obtained in step 1. First, 2-bromobiphenyl (1.05 equivalents, 4.0 g, 16.5 mmol) is solubilised in 102 ml of anhydrous diethyl ether. This solution is cooled to -60°C and n-BuLi (1.16 eq.) is added dropwise. After 10 min at this temperature, a white precipitate appeared which was redissolved while the medium was warmed to room temperature. 3-Bromofluorenone was then added and the reaction mixture was kept at 45 °C for one night.

After addition of NH₄Cl (5% aq., 260 ml) and extraction with diethyl ether, 7.0 g of an alcohol was obtained. This solid was solubilised in 141 ml of acetic acid and hydrolyzed by the addition of 78 ml of HCl/dioxane (10 % mol., 20 eq.). After evaporation of the solvents, the solid was subjected to normal phase flash chromatography to afford 5.86 g of the target compound (94 % yield).

Step 3: 3-phenoxazyl -SBF

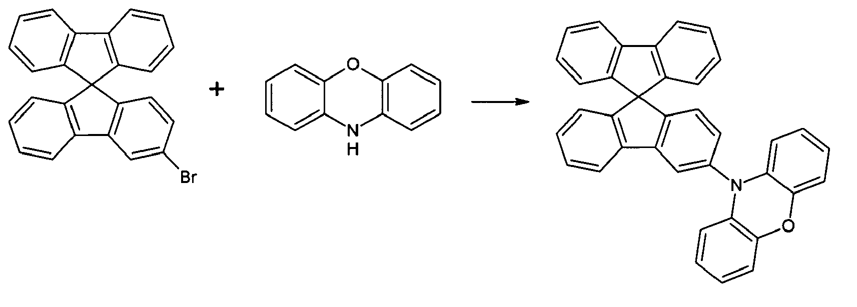

In a 500 ml flask, 320 ml of toluene were degassed during 2 hours. 3-Bromo SBF (9.9 g, 0.025 mol), sodium tert. butylate (tBuONa, 4.92 g, 0.05 mol) and phenoxazine (4,6g, 0.025 mol) were added and after 30 min, a mixture of Bis-dibenzylideneacetone palladium (Pd(dba)₂) and Tris-tert.-butylphosphine (P(tBu)₃) previously prepared was slowly added.

The solution was warmed to 110 °C for one hour and then filtrated on a celite pad. After evaporation of the solvent, the solid recovered was recrystallised in ethanol to afford 9.4 g of a white solid (77 % yield).

## Claims

1. Compounds of general formulae 1 to 12 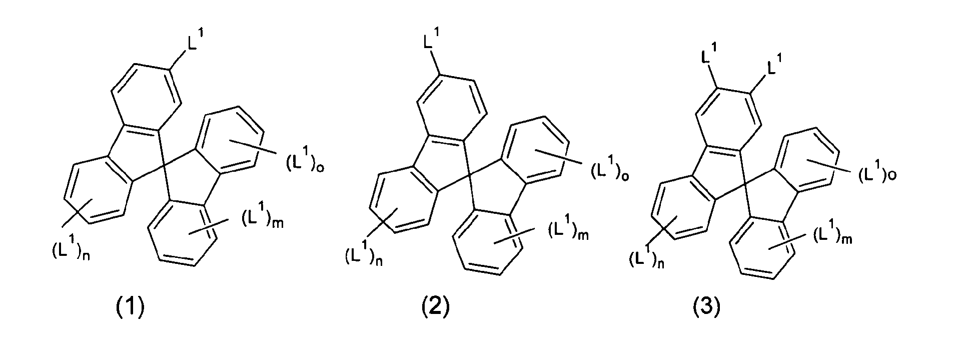 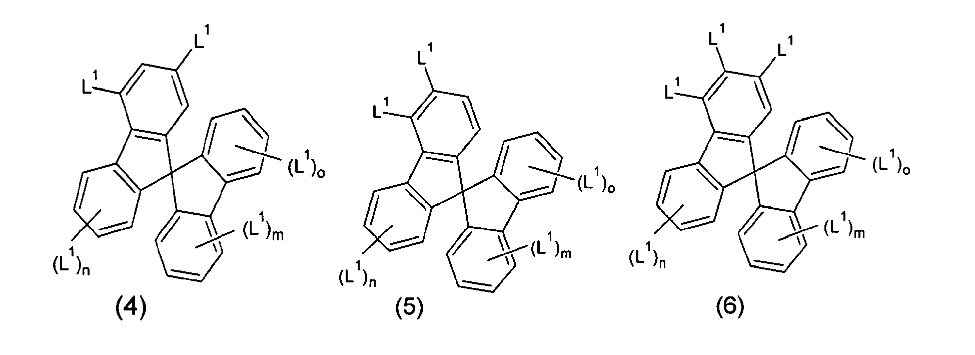 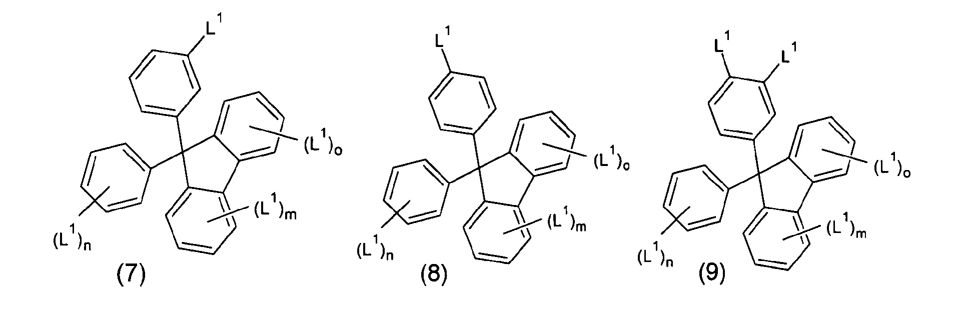 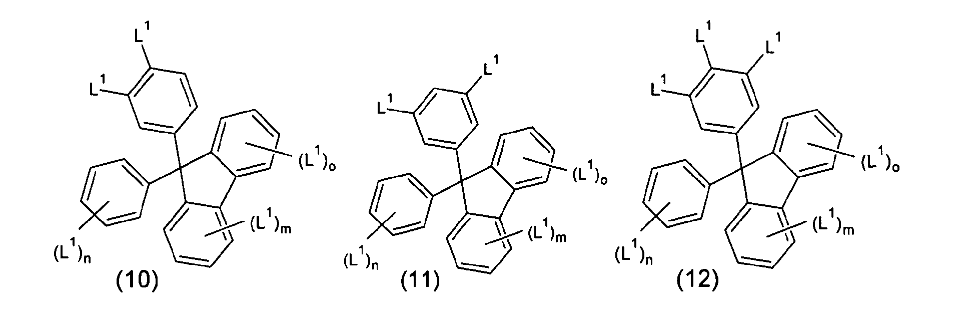 wherein n, m and o may be the same or different and represent an integer of from 0 to 3, with the proviso that for compounds of formula (1) and (7) at least one of n, m or o is zero,
each of the phenyl rings may carry no ligands other than L¹ or may be substituted by ligands other than L¹,
L¹, which may be the same or different at each position, has the formula A 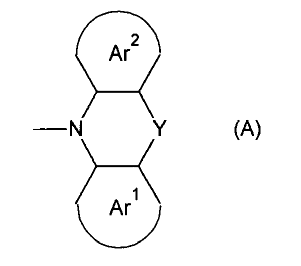 wherein
Y is selected from the group consisting of O, S, C=O, -CR¹R², N-R³, S=O, S(=O)₂, PR⁴ and P(=O)R⁵,
R¹ to R⁵ may be the same or different and represent hydrogen or an aliphatic, carbocyclic, aromatic or heterocyclic group with 1 to 20 carbon atoms, and Ar¹ and Ar², which may be the same or different, represent optionally substituted aromatic or heteroaromatic ring systems comprising 4 to 20 ring atoms.

2. Compounds in accordance with claim 1, wherein L¹ has the formula A-1 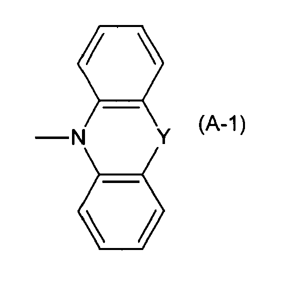 wherein Y has the meaning as defined in claim 1.

3. Compounds in accordance with at least one of claims 1 or 2 wherein Y is selected from the group consisting of O, S, C=O, CR¹R², N-R³ and S=O, wherein R¹ to R³ have the meaning as defined in claim 1.

4. Compounds in accordance with claim 3, wherein Y is selected from the group consisting of O, S and N-R³, wherein R³ has the meaning as defined in claim 1.

5. Compounds in accordance with claim 4, wherein Y is O or S, preferably O.

6. Compounds in accordance with at least one of claims 1 to 5 wherein at least one of n, m or o represents an integer of from 1 to 3.

7. Compounds in accordance with at least one of claims 1 to 5 wherein n, m and o are zero.

8. Use of the compounds in accordance with at least one of claims 1 to 7 in an organic light emitting device.

9. The use of claim 8 wherein the light emitting device is an organic light emitting diode.

10. Use of the light emitting material according to at least one of claims 1 to 7 as host in an emissive layer.

11. An organic light emitting device (OLED) comprising an emissive layer (EML), said emissive layer comprising a compound according to at least one of claims 1 to 7, with an emitting material.
